# EUROPEAN PATENT APPLICATION

(11) **EP 3 889 170 A1**
(43) Date of publication of application: **06.10.2021**
(21) Application number: 19890696.8
(22) Date of filing: 25.11.2019
(51) Int. Cl.: C07K 14/47, C07K 14/76, C07K 16/00, C07K 16/42, C07K 16/44, C07K 17/00, G01N 33/68, G01N 33/70, G01N 33/72, G01N 33/74

(54) **BIOMARKER FOR DIAGNOSING AT-RISK MENTAL STATE**

(30) Priority: 29.11.2018 JP 2018223521
(71) Applicant: Resvo Inc., Kawasaki-shi, Kanagawa 210-0007 (JP)
(72) Inventor: OHNISHI Arata, Izumo-shi Shimane 693-0021 (JP)
(74) Representative: Maikowski & Ninnemann Patentanwälte Partnerschaft mbB
(86) International application number: PCT/JP2019/045928
(87) International publication number: WO 2020/110982

(57) **Abstract**

The inventors discovered that in a sample from ARMS patients, the amounts of biopyrrin and cortisol significantly increase as well as the amounts of an immunoglobulin free κ chain (κFLC) and an immunoglobulin free λ chain (λFLC) significantly decrease. Therefore, the invention provides a biomarker for diagnosing ARMS comprising one or more selected from the group consisting of biopyrrin, cortisol, a κFLC or a fragment thereof, and a λFLC or a fragment thereof. By measuring the amount of the biomarker for diagnosing ARMS of the invention in a biological sample, ARMS diagnosis of a subject can be performed quickly, easily and accurately.

## Description

### Technical Field

The invention relates to a biomarker for diagnosing at-risk mental state (ARMS). The invention also relates to a kit for diagnosing ARMS, comprising a substance capable of binding to the biomarker for diagnosing ARMS, and a method for diagnosing ARMS, comprising a step of measuring the amount of the biomarker for diagnosing ARMS in a biological sample.

### Background Art

ARMS means a state at high risk of entering into the development of psychiatric disorders. Approximately 20 to 30 % of ARMS patients develop a variety of psychiatric disorders within a few years. The early detection and treatment of psychiatric disorders are currently regarded as important, and hence it is required to detect ARMS quickly and accurately. However, ARMS shows mild and gentle symptoms outwardly, and has a problem that it is very difficult to distinguish ARMS from a typical depression state without a careful examination.

Attempts have also been previously made to search for biomarkers for diagnosing psychiatric disorders (e. g. Patent Literatures 1 to 3). On the other hand, few attempts have been made for the diagnosis of ARMS. Therefore, the inventors of the invention set out to develop a new diagnostic method for ARMS with the aim of early detection of ARMS.

### Citation List

### Patent Literature

Patent Literature 1: Japanese Patent Laid-Open No. 2017-215335
Patent Literature 2: Japanese Patent Laid-Open No. 2017-114855
Patent Literature 2: Japanese Translation of PCT Application No. 2015-019979

### Non-patent Literature

Non-patent Literature 1: Bun Chino, "Medical examination for ARMS in a clinic for diagnosis and treatment," Japanese Journal of Preventive Psychiatry, Vol.1 (1) 2016, p.37-47.

### Summary of Invention

### Technical Problem

An object of the invention is to provide a biomarker for diagnosing ARMS, a kit for diagnosing ARMS, comprising a substance capable of binding to the biomarker for diagnosing ARMS, and a method for diagnosing ARMS, comprising a step of measuring the amount of the biomarker for diagnosing ARMS in a biological sample.

### Solution to Problem

In view of the above-described problems, the present inventors conducted studies. The inventors have compared and investigated immune substances and stress-related substances which are present in biological samples from ARMS patients and those from healthy subjects. As a result, the inventors have discovered that, regarding ARMS patients, the amounts of biopyrrin and cortisol are significantly increased compared with those of healthy subjects, and the amounts of an immunoglobulin free κ chain (κFLC), and an immunoglobulin free λ chain (λFLC) are significantly decreased compared with those of the healthy subjects. Namely, one or more biological substances selected from the group consisting of biopyrrin, cortisol, a κFLC or a fragment thereof, and a λFLC or a fragment thereof can be used as a biomarker for diagnosing ARMS.

Accordingly, the invention provides a biomarker for diagnosing at-risk mental state (ARMS), comprising one or more selected from the group consisting of biopyrrin, cortisol, an immunoglobulin free κ chain (κFLC) or a fragment thereof, and an immunoglobulin free λ chain (λFLC) or a fragment thereof. The biomarker for diagnosing ARMS may further comprise one or more selected from the group consisting of creatinine and albumin.

The invention also provides a kit for diagnosing at-risk mental state (ARMS), comprising one or more substances selected from the group consisting of a substance capable of binding to biopyrrin, a substance capable of binding to cortisol, a substance capable of binding to an immunoglobulin free κ chain (κFLC) or a fragment thereof, and a substance capable of binding to an immunoglobulin free λ chain (λFLC) or a fragment thereof. The kit for diagnosing ARMS may further comprise one or more substances selected from the group consisting of a substance capable of binding to creatinine and a substance capable of binding to albumin. In one embodiment, the substance is an antibody or an organic compound. In one embodiment, the substance is immobilized on a solid-support body.

The invention further provides a method for diagnosing at-risk mental state (ARMS) in a subject or a method for obtaining data for diagnosing ARMS in a subject, comprising a step of measuring one or more amounts selected from the group consisting of the amount of biopyrrin, the amount of cortisol, the amount of an immunoglobulin free κ chain (κFLC) or a fragment thereof, and the amount of an immunoglobulin free λ chain (λFLC) or a fragment thereof in a biological sample collected from the subject. The method of the invention may further comprise a step of measuring the amount of albumin in the biological sample. The method of the invention may further comprise a step of measuring the amount of creatinine in the biological sample. In one embodiment, the method of the invention may further comprise a step of comparing the amount with that of a control. In one embodiment, the method of the invention may further comprise a step of calculating a ratio value of an amount thereof to the amount of creatinine in the biological sample. In this embodiment, the method of the invention may further comprise a step of comparing the calculated ratio value with that of a control. In one embodiment, the biological sample is urine.

### Advantageous Effects of Invention

The biomarker for diagnosing ARMS according to the invention can be used to diagnose ARMS in a subject quickly, easily, and accurately. Further, the biomarker for diagnosing ARMS according to the invention can be detected in a urine sample from a subject. Therefore, the method of the invention is non-invasive.

### Brief Description of Drawings

[Figure 1] Figure 1 shows ROC (Receiver Operating Characteristic) curves for four factors that showed significant differences in Example 2.
[Figure 2] Figure 2 shows ROC curves after logistic regression analysis for four factors that showed significant differences in Example 2.

### Description of Embodiments

### (1. Definitions)

As used herein, the term "psychiatric disorder" has a meaning in the broadest sense in the art of the invention. Examples of the term "psychiatric disorder" include but not limited to, schizophrenia, autism, Alzheimer's disease, cognitive abnormality, depression, bipolar disorder (manic-depressive psychosis), neurodevelopmental disorder with anomalies of the brain, cognitive impairment ascribed to neuropathy due to infection during pregnancy, mental disorder ascribed to impaired immunity, epilepsy, idiophrenic insanity, toxic psychosis, intellectual disability (mental retardation), psychopathy, neurosis, psychiatric disorder ascribed to syphilitic infection, senile psychiatric disorders, psychiatric disorders after a cerebrovascular accident, psychiatric disorders due to a head injury, atypical endogenous psychosis, endocrine and extrinsic psychiatric disorders, involutional psychosis, and the like.

As used herein, the term "at-risk mental state " has a meaning in the broadest sense in the art of the invention. For example, the term "at-risk mental state " means a state at high risk of entering into the development (onset) of psychiatric disorders. At-risk mental state is a prodromal state of psychiatric disorders, different from a state of psychiatric disorders, for example, schizophrenia or autism. In general, approximately 20 to 30 % of patients with at-risk mental state develop a variety of psychiatric disorders within a few years. A subject with at-risk mental state does not always develop a particular psychiatric disorder. It is not sure what psychiatric disorder a subject with at-risk mental state will develop until it actually develops. As used herein, the term "at-risk mental state" is also referred to as "ARMS" (At-Risk Mental State).

As used herein, the term "diagnosis" has a meaning in the broadest sense in the art of the invention. Each term of "diagnosis of at-risk mental state", "diagnosing at-risk mental state", "diagnosis of ARMS", or "diagnosing ARMS " means, for example, determining whether or not a subject has ARMS, has potential ARMS, or is cured of ARMS or staying in remission of ARMS, or predicting the prognosis of ARMS in a subject.

As used herein, the term "biomarker for diagnosing at-risk mental state" means a substance capable of being an indicator for ARMS diagnosis, e.g. a biological substance capable of being an indicator for ARMS diagnosis. As used herein, the term "biomarker for diagnosing at-risk mental state" is also referred to as "biomarker for diagnosing ARMS." By measuring the amount of a biomarker for diagnosing ARMS in a biological sample collected from a subject, and comparing the amount with that of a control, ARMS diagnosis of the subject can be made. The term "biomarker for diagnosing at-risk mental state" may comprise one or a plurality (e.g. 2, 3, 4, 5 or 6) of biomarkers. For example, when the amounts of 2 or more certain biological substances increase or decrease in an ARMS patient in comparison with those in healthy subjects, the combination of these 2 or more biological substances can be the biomarker for diagnosing ARMS. Also, for example, when the amounts of 2 or more certain biological substances increase or decrease in a patient with a certain disease or condition other than ARMS in comparison with those in healthy subjects, while, in an ARMS patient, the amounts of 1 or more of them increase or decrease, and the amounts of the others do not change in comparison with those in healthy subjects, the combination of these 2 or more biological substances can be the biomarker for diagnosing ARMS. When the biomarker for diagnosing ARMS comprises a plurality of biomarkers, the reliability of ARMS diagnosis may be enhanced.

As used herein, the term "biological substance" has a meaning in the broadest sense in the art of the invention. For example, the term "biological substance" is a chemical substance present in a body of a subject or a sample collected from a subject. The term "biological substance" includes chemical substances present in a biological sample, such as small molecules, large molecules, peptides, polypeptides, proteins, or antibodies or fragments thereof. Biopyrrin, cortisol, an immunoglobulin free κ chain (κFLC) or a fragment thereof, an immunoglobulin free λ chain (λFLC) or a fragment thereof, creatinine, or albumin can be the biological substance.

The term "biopyrrin" is a substance known in the art of the invention, and is the final oxidation product produced when bilirubin reacts with active oxygen in vivo. The term "biopyrrin" is also known as an oxidative stress marker in urine. As used herein, the term "biopyrrin" includes those that are modified in vivo, such as those that are phosphorylated, sugar-chain added (glycosylated), amidated, ubiquitinated, and/or acetylated.

The term "cortisol" is a substance known in the art of the invention, and is a kind of glucocorticoid which is an adrenocortical hormone. The term "cortisol" is also known as a psychological stress marker. As used herein, the term "cortisol" includes those that are modified in vivo, such as those that are phosphorylated, sugar-chain added (glycosylated), amidated, ubiquitinated, and/or acetylated.

The term "immunoglobulin free κ chain" and the term "immunoglobulin free λ chain" are used generally in the art of the invention, which mean a κ immunoglobulin chain in free form and a λ immunoglobulin chain in free form, respectively. The term "free" means being present alone without expression within IgG or on B cells or the like. As used herein, the immunoglobulin free κ chain is also referred to as "κFLC" (kappa free light chain), and the immunoglobulin free λ chain is also referred to as "λFLC" (lambda free light chain). As used herein, the term "immunoglobulin free κ chain" and the term "immunoglobulin free λ chain" include those that are modified in vivo, such as those that are phosphorylated, sugar-chain added (glycosylated), amidated, ubiquitinated, and/or acetylated.

The term "creatinine" is a substance known in the art of the invention. The term "creatinine" is also known as a substance used in a renal function test. As used herein, the term "creatinine" includes those that are modified in vivo, such as those that are phosphorylated, sugar-chain added (glycosylated), amidated, ubiquitinated, and/or acetylated.

The term "albumin" is also a substance known in the art of the invention. As used herein, the term "albumin" includes those that are modified in vivo, such as those that are phosphorylated, sugar-chain added (glycosylated), amidated, ubiquitinated, and/or acetylated.

In the context of the biomarker, the term "fragment" means a polypeptide consisting of a partial amino acid sequence of a full-length protein. For example, the term "fragment" refers to a polypeptide having amino acid residues of at least one amino acid residue shorter than the full-length protein, e.g. a polypeptide having amino acid residues of about 1 to about 100, about 1 to about 50, about 1 to about 20, about 1 to about 10, about 1 to about 5 amino acid residue shorter than the full-length protein. For example, the fragment of κFLC is a constant region thereof (immunoglobulin κ chain C region) or a site capable of being an antigen in detection with an antibody (antigen site). For example, the fragment of AFLC is a constant region thereof (immunoglobulin λ chain C region) or a site capable of being an antigen in detection with an antibody (antigen site). The term "fragment" may be a single or a mixture consisting of multiple species.

As used herein, the term "subject" has a meaning in the broadest sense in the art of the invention. The term "subject" includes human or non-human animals. The non-human animals include non-human mammals such as rats, mice, guinea pigs, rabbits, monkeys, dogs, cats, or miniature pigs. When the subject is a human, the subject may be referred to as "examinee" or "patient." When the subject is a human, the subject may be a male or a female. When the subject is a human, the subject may include, but not limited to, a neonate, suckling infant, infant, juvenile (boy or girl), sub-adult (young-adult), prime-age adult, middle-aged adult, older-adult, and the like.

As used herein, the term "biological sample" has a meaning in the broadest sense in the art of the invention. Examples of the term "biological sample" include a body fluid such as blood or urine, or a cell. The term "blood" includes serum, plasma, and whole blood. The biomarker for diagnosing ARMS of the invention may be present in a biological sample.

In the present specification, the term "measurement" has a meaning in the broadest sense in the art of the invention. As used herein, the term "measurement" includes quantification. The term "quantification" includes semi-quantification. The unit of amount (quantity) includes, but not limited to, mg, µg, ng, mmol, pmol, nmol, and the like. In the context of the biomarker for diagnosing ARMS of the invention, the term "amount" includes the concentration. The unit of the concentration includes, but not limited to, mg/ml, pg/ml, ng/ml, mmol/ml, pmol/ml, nmol/ml, and the like. In the context of the biomarker for diagnosing ARMS of the invention, the term "amount" may be a ratio to a particular substance, e.g. an internal standard substance. In that case, the unit of amount includes, but is not limited to, mg/g, pg/g, ng/g, mmol/g, pmol/g, nmol/g and the like (ratio to the amount of an internal standard substance).

In certain embodiments, the term "amount" includes radiation intensity, fluorescence intensity, or absorbance of a labeling marker bound to the biomarker for diagnosing ARMS of the invention. In further certain embodiments, the term "amount" is the amount of any secondary antibodies that bind to any primary antibodies that are bound to the biomarker for diagnosing ARMS of the invention. The term "amount" may be an absolute amount or a relative amount. In certain embodiments, the amount value of the biomarker for diagnosing ARMS of the invention is corrected by the amount value of a specific substance. The specific substance is, for example, a substance in a biological sample such as creatinine. The corrected value is a value that is added, subtracted, multiplied, or divided (ratio value) with an arbitrary value, for example, a reference value, an amount value of a specific substance, or the like.

As used herein, the term "control" has a meaning in the broadest sense in the art of the invention. For example, the term "control" means a subject to be compared with the subject who is diagnosed, by using the biomarker for diagnosing ARMS of the invention, by using the kit of the invention or via the method of the invention. Of the control, the amount of the biomarker for diagnosing ARMS of the invention may also be measured according to the method of the invention. The term "control" includes, for example, a normal subject, a healthy subject, a subject not diagnosed with ARMS, a subject who has been previously diagnosed with ARMS but is currently cured or ameliorated, a subject before or after administration of a candidate drug for treating ARMS or psychiatric disorders, or the subject oneself in the past. The control may be the same person as the subject or another person.

As used herein, the term "about" means that there may be a variable range that can be tolerated by a person skilled in the art, e.g. a variable range of ± 0.1 to 20%, ± 0.1 to 10%, ± 0.1 to 5%, ± 0.1 to 1.0%, or ±0.1 to 0.5%.

### (2. The biomarker for diagnosing ARMS of the invention)

The biomarker for diagnosing ARMS of the invention comprises one or more (e.g., 1, 2, 3, 4, 5 or 6) selected from the group consisting of biopyrrin, cortisol, an immunoglobulin free κ chain (κFLC) or a fragment thereof, and an immunoglobulin free λ chain (λFLC) or a fragment thereof. In a particular embodiment, the biomarker for diagnosing ARMS of the invention consists of one or more (e.g., 1, 2, 3, 4, 5 or 6) selected from the group consisting of biopyrrin, cortisol, an immunoglobulin free κ chain (κFLC) or a fragment thereof, and an immunoglobulin free λ chain (λFLC) or a fragment thereof. Biopyrrin, cortisol, a κFLC or a fragment thereof, and/or a λFLC or a fragment thereof may be modified in vivo, such as phosphorylated, sugar-chain added (glycosylated), amidated, ubiquitinated, and/or acetylated.

In a preferred embodiment, the biomarker for diagnosing ARMS of the invention comprises biopyrrin. In another embodiment, the biomarker for diagnosing ARMS of the invention comprises cortisol. In another embodiment, the biomarker for diagnosing ARMS of the invention comprises a κFLC or a fragment thereof. In another embodiment, the biomarker for diagnosing ARMS of the invention comprises a λFLC or a fragment thereof.

In one embodiment, the biomarker for diagnosing ARMS of the invention comprises a combination of 2 or more (e.g., 2, 3, 4, 5 or 6) selected from the group consisting of biopyrrin, cortisol, a κFLC or a fragment thereof, and a λFLC or a fragment thereof. In a specific embodiment, the biomarker for diagnosing ARMS of the invention consists of a combination of 2 or more (e.g., 2, 3, 4, 5 or 6) selected from the group consisting of biopyrrin, cortisol, a κFLC or a fragment thereof, and a λFLC or a fragment thereof. By use of the combination of 2 or more selected from the group consisting of biopyrrin, cortisol, a κFLC or a fragment thereof, and a λFLC or a fragment thereof, the reliability of the ARMS diagnosis can be enhanced.

In a preferred embodiment, the biomarker for diagnosing ARMS of the invention comprises biopyrrin; and one or more (e.g., 1, 2, 3, 4, 5 or 6) selected from the group consisting of cortisol, a κFLC or a fragment thereof, and a λFLC or a fragment thereof. In other embodiments, the biomarker for diagnosing ARMS of the invention comprises cortisol; and one or more (e.g., 1, 2, 3, 4, 5 or 6) selected from the group consisting of biopyrrin, a κFLC or a fragment thereof, and a λFLC or a fragment thereof. In other embodiments, the biomarker for diagnosing ARMS of the invention comprises, a κFLC or a fragment thereof; and one or more (e.g., 1, 2, 3, 4, 5 or 6) selected from the group consisting of biopyrrin, cortisol, and a λFLC or a fragment thereof. In further other embodiments, the biomarker for diagnosing ARMS of the invention comprises, a λFLC or a fragment thereof; and one or more (e.g., 1, 2, 3, 4, 5 or 6) selected from the group consisting of biopyrrin, cortisol, and a κFLC or a fragment thereof.

In one embodiment, the biomarker for diagnosing ARMS of the invention comprises biopyrrin and cortisol. In a preferred embodiment, the biomarker for diagnosing ARMS of the invention comprises biopyrrin and a κFLC or a fragment thereof. In other preferred embodiments, the biomarker for diagnosing ARMS of the invention comprises biopyrrin and a λFLC or a fragment thereof. In other embodiments, the biomarker for diagnosing ARMS of the invention comprises cortisol and a κFLC or a fragment thereof. In further other embodiments, the biomarker for diagnosing ARMS of the invention comprises cortisol and a λFLC or a fragment thereof. In furthermore other embodiments, the biomarker for diagnosing ARMS of the invention comprises a κFLC or a fragment thereof, and a λFLC or a fragment thereof.

In one embodiment, the biomarker for diagnosing ARMS of the invention comprises biopyrrin, cortisol, and a κFLC or a fragment thereof. In other embodiments, the biomarker for diagnosing ARMS of the invention comprises biopyrrin, cortisol, and a λFLC or a fragment thereof. In a preferred embodiment, the biomarker for diagnosing ARMS of the invention comprises biopyrrin, a κFLC or a fragment thereof and, a λFLC or a fragment thereof. In further other embodiments, the biomarker for diagnosing ARMS of the invention comprises cortisol, a κFLC or a fragment thereof, and a λFLC or a fragment thereof. In furthermore other embodiments, the biomarker for diagnosing ARMS of the invention comprises biopyrrin, cortisol, a κFLC or a fragment thereof, and a λFLC or a fragment thereof.

The structure of biopyrrin is known. Biopyrrin in a biological sample can be detected, measured or compared by a known method. For example, biopyrrin in a biological sample can be detected, measured or compared by use of Biopyrrin ELISA kit (Metallogenics Co., Ltd). Also, biopyrrin in a biological sample can be detected, measured or compared by use of e.g., the kit for diagnosing ARMS of the invention. In ARMS patients, the amount of biopyrrin increases.

The structure of cortisol is known. Cortisol in a biological sample can be detected, measured or compared by a known method. For example, cortisol in a biological sample can be detected, measured or compared by use of DetectX CORTISOL Enzyme Immunoassay Kit (ARBOR ASSAYS Inc.). Also, cortisol in a biological sample can be detected, measured or compared by use of e.g., the kit for diagnosing ARMS of the invention. In ARMS patients, the amount of cortisol increases.

The amino acid sequence of κFLC is known. The κFLC or fragment thereof in a biological sample can be detected, measured or compared by a known method. For example, the κFLC or fragment thereof in a biological sample can be detected, measured or compared by use of FREELITE κ chain (MBL Co., Ltd., Code No.: BS-LK016BD). Also, the κFLC or fragment thereof in a biological sample can be detected, measured or compared by use of e.g., the kit for diagnosing ARMS of the invention. In ARMS patients, the amount of the κFLC or fragment thereof decreases.

The κFLC may comprise the constant region (immunoglobulin κ chain C region) and/or the antigen site thereof. The fragment of κFLC may be a constant region (immunoglobulin κ chain C region) or an antigen site of the κFLC. The κFLC may be human-derived or non-human-derived, such as rats, mice or rabbits. In one embodiment, the κFLC comprises the amino acid sequence of SEQ ID NO:1 below (immunoglobulin κ chain C region) (human-derived).

### [Formula 1]

Thr Val Ala Ala Pro Ser Val Phe Ile Phe Pro Pro Ser Asp Glu Gln Leu Lys Ser Gly Thr Ala
Ser Val Val Cys Leu Leu Asn Asn Phe Tyr Pro Arg Glu Ala Lys Val Gln Trp Lys Val Asp Asn
Ala Leu Gln Ser Gly Asn Ser Gln Glu Ser Val Thr Glu Gln Asp Ser Lys Asp Ser Thr Tyr Ser
Leu Ser Ser Thr Leu Thr Leu Ser Lys Ala Asp Tyr Glu Lys His Lys Val Tyr Ala Cys Glu Val
Thr His Gln Gly Leu Ser Ser Pro Val Thr Lys Ser Phe Asn Arg Gly Glu Cys (SEQ ID NO:1)

Preferably, the κFLC comprises the amino acid sequence of SEQ ID NO: 2 or 3 below (antigen site) (human-derived).

### [Formula 2]

Ser Val Thr Glu Gln Asp Ser Lys Asp Ser Thr Tyr Ser Leu Ser Ser Thr Leu Thr Leu (SEQ ID NO:2); or
Cys Glu Val Thr His Gln Gly Leu Ser Ser Pro Val Thr Lys Ser Phe Asn Arg Gly Glu Cys (SEQ ID NO:3)

In other embodiments, the κFLC comprises the amino acid sequence of SEQ ID NO: 4 below (immunoglobulin κ chain C region) (rat-derived).

### [Formula 3]

Ala Asp Ala Ala Pro Thr Val Ser Ile Phe Pro Pro Ser Met Glu Gln Leu Thr Ser Gly Gly Ala
Thr Val Val Cys Phe Val Asn Asn Phe Tyr Pro Arg Asp Ile Ser Val Lys Trp Lys Ile Asp Gly
Ser Glu Gln Arg Asp Gly Val Leu Asp Ser Val Thr Asp Gln Asp Ser Lys Asp Ser Thr Tyr Ser
Met Ser Ser Thr Leu Ser Leu Thr Lys Val Glu Tyr Glu Arg His Asn Leu Tyr Thr Cys Glu Val
Val His Lys Thr Ser Ser Ser Pro Val Val Lys Ser Phe Asn Arg Asn Glu Cys (SEQ ID NO:4)

Preferably, the κFLC comprises the amino acid sequence of SEQ ID NO: 5 below (antigen site) (rat-derived).

### [Formula 4]

Ser Val Thr Asp Gln Asp Ser Lys Asp Ser Thr Tyr Ser Met Ser Ser Thr Leu Ser Leu (SEQ ID NO:5)

The amino acid sequence of λFLC is known. The λFLC or fragment thereof in a biological sample can be detected, measured or compared by a known method. For example, the λFLC or fragment thereof in a biological sample can be detected, measured or compared by use of FREELITE λ chain (MBL Co., Ltd., Code No.: BS-LK018BD). Also, the λFLC in a biological sample can be detected, measured or compared by use of e.g., the kit for diagnosing ARMS of the invention. In ARMS patients, the amount of the λFLC or fragment thereof decreases.

The λFLC may comprise the constant region (immunoglobulin λ chain C region) and/or the antigen site thereof. The fragment of λFLC may be a constant region of (immunoglobulin κ chain C region) or an antigen site of the λFLC. The λFLC may be human-derived or non-human derived, such as rats, mice, or rabbits. In one embodiment, the λFLC comprises the amino acid sequence of SEQ ID NO:6 below (immunoglobulin λ chain C region) (human-derived).

### [Formula 5]

Ala Pro Ser Val Thr Leu Phe Pro Pro Ser Ser Glu Glu Leu Gln Ala Asn Lys Ala Thr Leu Val
Cys Leu Ile Ser Asp Phe Tyr Pro Gly Ala Val Thr Val Ala Trp Lys Ala Asp Ser Ser Pro Val
Lys Ala Gly Val Glu Thr Thr Thr Pro Ser Lys Gln Ser Asn Asn Lys Tyr Ala Ala Ser Ser Tyr
Leu Ser Leu Thr Pro Glu Gln Trp Lys Ser His Lys Ser Tyr Ser Cys Gln Val Thr His Glu Gly
Ser Thr Val Glu Lys Thr (SEQ ID NO:6)

A κFLC and λFLC comprising an amino acid sequence derived from non-human such as rats can be used to diagnose ARMS in an ARMS model animal. It is useful, particularly, for the preparation, quality control, or the like of ARMS model animals.

The κFLC or λFLC having the amino acid sequence with deletion, addition, variation, and/or substitution therein can be within the scope of the invention, provided that the uniqueness of κFLC or λFLC, e.g., an amino acid sequence characterizing κFLC or λFLC (e.g., a motif, a domain, a box sequence, or the like), or the like, is retained. The number of such deletions, additions, variations, and/or substitutions is not specially limited (e.g., 1, 2, 3, 4, 5, 6, 7, 8, 9, 10, or more) provided that the uniqueness of κFLC or λFLC is retained. In general, variation or substitution between amino acids having the same properties has no or little effect on the function of κFLC or λFLC; thus, κFLC or λFLC containing such variation or substitution can be equated with the original κFLC or λFLC (e.g., the κFLC or λFLC specified by any of the above SEQ ID NOs). The properties of such amino acids can be categorized, for example, as follows: acidic amino acids (aspartic acid and glutamic acid), basic amino acids (lysine, histidine, and arginine), hydrophobic (aliphatic) amino acids (glycine, alanine, valine, leucine, and isoleucine), sulfur-containing amino acids (methionine and cysteine), aromatic amino acids (phenylalanine, tryptophan, and tyrosine), acidic amino acid amides (asparagine and glutamine), or neutral amino acids (glycine, alanine, valine, leucine, isoleucine, phenylalanine, tyrosine, tryptophan, proline, serine, threonine, cysteine, methionine, asparagine, and glutamine). For example, with regard to the amino acid sequence of SEQ ID NO: 1, the amino acid sequence in which Glu at the 105th position and Cys at the 106th position are deleted is also included within the scope of the invention.

In addition to the above, amino acid sequences having a certain sequence identity (e.g., at least about 70 % identity, at least about 80 % identity, at least about 85 % identity, at least about 90 % identity, at least about 95 % identity, and at least about 99 % identity) to the amino acid sequence of κFLC or λFLC are also included within the scope of the invention, provided that the uniqueness of κFLC or λFLC, e.g., an amino acid sequence characterizing κFLC or λFLC (e.g., a motif, a domain, a box sequence, or the like), or the like, is retained.

The biomarker for diagnosing ARMS of the invention may further comprise one or more selected from the group consisting of creatinine and albumin. In one embodiment, the biomarker for diagnosing ARMS of the invention further comprises creatinine. In other embodiments, the biomarker for diagnosing ARMS of the invention further comprises albumin. In other embodiments, the biomarker for diagnosing ARMS of the invention further comprises creatinine and albumin.

Urine collected from a subject may be concentrated or diluted caused by diurnal variation. Due to the diurnal variation, the concentrations of biopyrrin, cortisol, a κFLC or a fragment thereof, and/or a λFLC or a fragment thereof may change. In order to compensate for the effects of these variations, creatinine can be used as an internal standard substance. The amounts of biopyrrin, cortisol, a κFLC or a fragment thereof, and/or a λFLC or a fragment thereof in a biological sample collected from a subject can be corrected by using the amount of creatinine in the biological sample. Preferably, each of the concentrations of biopyrrin, cortisol, a κFLC or a fragment thereof, and/or a λFLC or a fragment thereof in a biological sample collected from a subject is corrected by the concentration of creatinine in the biological sample.

In addition, by correcting the amounts of biopyrrin, cortisol, a κFLC or a fragment thereof, and/or a λFLC or a fragment thereof in a biological sample collected from a subject by the amount of creatinine, a value reflecting the renal function of the subject can be obtained. By comparing the corrected value with that of a control, ARMS can be diagnosed with higher accuracy. The correction may include division, namely calculating a ratio value thereof to the amount of creatinine.

Further, creatinine and/or albumin may be used to conclude if there is any abnormality in renal function. If renal function is abnormal, some of the amount of biopyrrin, the amount of cortisol, the amount of a κFLC or a fragment thereof, and/or the amount of a λFLC or a fragment thereof may change even if the subject is not ARMS. For example, a subject having an abnormal renal function may show an increased amount of biopyrrin, increased amount of cortisol, decreased amount of a κFLC or a fragment thereof, and/or decreased amount of a λFLC or a fragment thereof, together with a changed (increased or decreased) amount of creatinine and/or albumin. In contrast, an ARMS patient with a normal renal function may show an increased amount of biopyrrin, increased amount of cortisol, decreased amount of a κFLC or a fragment thereof, and/or decreased amount of a λFLC or a fragment thereof, while showing the amount of creatinine and/or albumin may remain unchanged. From the above, a combination of one or more selected from the group consisting of biopyrrin, cortisol, a κFLC or a fragment thereof, and a λFLC or a fragment thereof, and, creatinine and/or albumin, can be used as a biomarker for diagnosing ARMS. Moreover, the amounts of creatinine and/or albumin may be used for determining whether the change in any of the amount of biopyrrin, the amount of cortisol, the amount of a κFLC or a fragment thereof, and/or the amount of a λFLC or a fragment thereof are attributed to an abnormality of renal function or not. For example, when the ratio of the amount of albumin/the amount of creatinine is greater than about 20, about 25, about 30, about 35, or about 40 mg/g, especially greater than about 30 mg/g, it can be concluded that the change in any of the amount of biopyrrin, the amount of cortisol, the amount of a κFLC or a fragment thereof, and/or the amount of a λFLC or a fragment thereof, is attributed to an abnormality of renal function. On the contrary, when the ratio of the amount of albumin/the amount of creatinine is about 5, about 10, about 15, about 20, about 25, about 30, about 35, or about 40 mg/g or less, especially, about 30 mg/g or less, it can be concluded that the change in any of the amount of biopyrrin, the amount of cortisol, the amount of a κFLC or a fragment thereof, and/or the amount of a λFLC or a fragment thereof, is not attributed to an abnormality of renal function, namely, renal function is normal.

The structure of creatinine is known. Creatinine in a biological sample can be detected, measured or compared by a known method. For example, creatinine in a biological sample can be detected, measured or compared by use of DetectX Creatinine detection Kit (ARBOR ASSAYS Inc.). Creatinine in a biological sample can be detected, measured or compared by use of the kit for diagnosing ARMS of the invention.

The structure of albumin is also known. Albumin in a biological sample can be detected, measured or compared by a known method. For example, albumin in a biological sample can be detected, measured or compared by using Albumin (Human) ELISA Kit (Cayman Chemical Company). Albumin in a biological sample can be detected, measured or compared by use of the kit for diagnosing ARMS of the invention.

ARMS can be diagnosed by use of the biomarker for diagnosing ARMS of the invention. Specifically, ARMS of a subject can be diagnosed by measuring the amount of the biomarker for diagnosing ARMS of the invention in a biological sample collected from the subject. The diagnosis of ARMS can be performed according to the method for diagnosing ARMS of the invention. Further, by use of the biomarker for diagnosing ARMS of the invention, data for diagnosing ARMS can be obtained. Specifically, by measuring the amount of the biomarker for diagnosing ARMS of the invention being present in a biological sample collected from a subject, data for diagnosing the subject with ARMS can be obtained. The method for obtaining data can be performed according to a method for obtaining data for diagnosing ARMS of the invention. Furthermore, by use of the biomarker for diagnosing ARMS of the invention, a substance capable of specifically binding to the biomarker for diagnosing ARMS of the invention (e.g., an antibody) can be prepared. By use of such a substance, the biomarker for diagnosing ARMS of the invention can be detected or measured.

Via the ARMS diagnosis using the biomarker for diagnosing ARMS of the invention, the screening of an ARMS patient, the establishment of an efficient therapeutic method for ARMS, the establishment of an efficient preventing method against the development of psychiatric disorders, the screening of a candidate agent useful in the ARMS treatment, and the development of an animal model of ARMS, can be performed.

### (3.Kit for diagnosing ARMS of the invention)

The kit for diagnosing ARMS of the invention comprises a substance capable of binding to the biomarker for diagnosing ARMS of the invention. The substance binds to the biomarker for diagnosing ARMS of the invention, whereby the biomarker for diagnosing ARMS of the invention can be detected, or the amount of the biomarker for diagnosing ARMS of the invention can be measured. The substance capable of binding to the biomarker for diagnosing ARMS of the invention may be one or a plurality (e.g., 2, 3, 4, 5 or 6) of substances. The substance capable of binding to the biomarker for diagnosing ARMS of the invention includes a substance capable of binding to biopyrrin, a substance capable of binding to cortisol, a substance capable of binding to an immunoglobulin free κ chain (κFLC) or a fragment thereof, a substance capable of binding to an immunoglobulin free λ chain (λFLC) or a fragment thereof, a substance capable of binding to creatinine, and/or a substance capable of binding to albumin. In one embodiment, the substance is capable of binding specifically to the biomarker for diagnosing ARMS of the invention. By using the substance capable of binding to the biomarker for diagnosing ARMS of the invention, the biomarker for diagnosing ARMS of the invention in a biological sample can be detected, and the amount of the biomarker for diagnosing ARMS of the invention can be measured.

In one embodiment, the kit for diagnosing ARMS of the invention may comprise one or more (e.g., 1, 2, 3, 4, 5, or 6) substances selected from the group consisting of a substance capable of binding to biopyrrin, a substance capable of binding to cortisol, a substance capable of binding to an immunoglobulin free κ chain (κFLC) or a fragment thereof, and a substance capable of binding to an immunoglobulin free λ chain (λFLC) or a fragment thereof.

In a preferred embodiment, the kit for diagnosing ARMS of the invention comprises a substance capable of binding to biopyrrin. In other embodiments, the kit for diagnosing ARMS of the invention comprises a substance capable of binding to cortisol. In another embodiment, the kit for diagnosing ARMS of the invention comprises a substance capable of binding to a κFLC or a fragment thereof. In further another embodiment, the kit for diagnosing ARMS of the invention comprises a substance capable of binding to a λFLC or a fragment thereof.

In a preferred embodiment, the kit for diagnosing ARMS of the invention comprises a substance capable of binding to biopyrrin; and one or more (e.g., 1, 2, 3, 4, 5 or 6) substances selected from the group consisting of a substance capable of binding to cortisol, a substance capable of binding to a κFLC or a fragment thereof, and a substance capable of binding to a λFLC or a fragment thereof. In another embodiment, the kit for diagnosing ARMS of the invention comprises a substance capable of binding to cortisol; and one or more (e.g., 1, 2, 3, 4, 5 or 6) substances selected from the group consisting of a substance capable of binding to biopyrrin, a substance capable of binding to a κFLC or a fragment thereof, and a substance capable of binding to a λFLC or a fragment thereof. In further another embodiment, the kit for diagnosing ARMS of the invention comprises a substance capable of binding to a κFLC or a fragment thereof; and one or more (e.g., 1, 2, 3, 4, 5 or 6) substances selected from the group consisting of a substance capable of binding to biopyrrin, a substance capable of binding to cortisol, and a substance capable of binding to a λFLC or a fragment thereof. In furthermore another embodiment, the kit for diagnosing ARMS of the invention comprises a substance capable of binding to a λFLC or a fragment thereof; and one or more (e.g., 1, 2, 3, 4, 5 or 6) substances selected from the group consisting of a substance capable of binding to biopyrrin, a substance capable of binding to cortisol, and a substance capable of binding to a κFLC or a fragment thereof.

In one embodiment, the kit for diagnosing ARMS of the invention comprises a substance capable of binding to biopyrrin and a substance capable of binding to cortisol. In a preferred embodiment, the kit for diagnosing ARMS of the invention comprises a substance capable of binding to biopyrrin and a substance capable of binding to a κFLC or a fragment thereof. In other preferred embodiments, the kit for diagnosing ARMS of the invention comprises a substance capable of binding to biopyrrin and a substance capable of binding to a λFLC or a fragment thereof. In another embodiment, the kit for diagnosing ARMS of the invention comprises a substance capable of binding to cortisol and a substance capable of binding to a κFLC or a fragment thereof. In further another embodiment, the kit for diagnosing ARMS of the invention comprises a substance capable of binding to cortisol and a substance capable of binding to a λFLC or a fragment thereof. In furthermore another embodiment, the kit for diagnosing ARMS of the invention comprises a substance capable of binding to a κFLC or a fragment thereof and a substance capable of binding to a λFLC or a fragment thereof.

In one embodiment, the kit for diagnosing ARMS of the invention comprises a substance capable of binding to biopyrrin, a substance capable of binding to cortisol, and a substance capable of binding to a κFLC or a fragment thereof. In other embodiments, the kit for diagnosing ARMS of the invention comprises a substance capable of binding to biopyrrin, a substance capable of binding to cortisol, and a substance capable of binding to a λFLC or a fragment thereof. In a preferred embodiment, the kit for diagnosing ARMS of the invention comprises a substance capable of binding to biopyrrin, a substance capable of binding to a κFLC or a fragment thereof, and a substance capable of binding to a λFLC or a fragment thereof. In another embodiment, the kit for diagnosing ARMS of the invention comprises a substance capable of binding to cortisol, a substance capable of binding to a κFLC or a fragment thereof, and a substance capable of binding to a λFLC or a fragment thereof. In further another embodiment, the kit for diagnosing ARMS of the invention comprises a substance capable of binding to biopyrrin, a substance capable of binding to cortisol, a substance capable of binding to a κFLC or a fragment thereof, and a substance capable of binding to a λFLC or a fragment thereof.

The kit for diagnosing ARMS of the invention may further comprise one or more substances selected from the group consisting of a substance capable of binding to creatinine and a substance capable of binding to albumin. In one embodiment, the kit for diagnosing ARMS of the invention further comprises a substance capable of binding to creatinine. In another embodiment, the kit for diagnosing ARMS of the invention further comprises a substance capable of binding to albumin. In furthermore another embodiment, the kit for diagnosing ARMS of the invention further comprises a substance capable of binding to creatinine and a substance capable of binding to albumin.

The substance capable of binding to the biomarker for diagnosing ARMS of the invention may include any substance having affinity to the biomarker for diagnosing ARMS of the invention. In one embodiment, the substance capable of binding to the biomarker for diagnosing ARMS of the invention is an antibody or organic compound. The organic compound may be a low-molecular-weight compound or a high-molecular-weight compound. Examples of the organic compound include a coloring matter, fluorochrome, dye, pigment, color reagent, coloring reagent, immunostaining reagent, enzyme, labeled compound and the like. The coloring reagent includes a compound that causes a color reaction with the biomarker for diagnosing ARMS of the invention. Examples of the labeled compound include, but not limited to, a compound labeled with an inorganic substance, coloring matter, fluorochrome, dye, pigment, color reagent, coloring reagent, immunostaining reagent, enzyme, radioisotope or the like. In one embodiment, any one of the substance capable of binding to biopyrrin, the substance capable of binding to cortisol, the substance capable of binding to a κFLC or a fragment thereof, the substance capable of binding to a λFLC or a fragment thereof, the substance capable of binding to creatinine, and/or the substance capable of binding to albumin, is an antibody or an organic compound. The antibody may include a polyclonal antibody or monoclonal antibody.

In one embodiment, the substance capable of binding to the biomarker for diagnosing ARMS of the invention is labeled with a labeling marker. In one embodiment, any one of the substance capable of binding to biopyrrin, the substance capable of binding to cortisol, the substance capable of binding to a κFLC or a fragment thereof, the substance capable of binding to a λFLC or a fragment thereof, the substance capable of binding to creatinine, and/or the substance capable of binding to albumin, is labeled with a labeling marker. Examples of the labeling marker include, but not limited to, an inorganic substance, coloring matter, fluorochrome, dye, pigment, color reagent, coloring reagent, immunostaining reagent, enzyme, radioisotope or the like. By detecting the labeling marker, the biomarker for diagnosing ARMS of the invention can be detected, and the amount of the biomarker for diagnosing ARMS of the invention can be measured

In the case that the substance capable of binding to the biomarker for diagnosing ARMS of the invention is an antibody, the antibody can be bound to the biomarker for diagnosing ARMS of the invention as a primary antibody, then, by using a secondary antibody, the biomarker for diagnosing ARMS of the invention can be detected, or the amount of the biomarker for diagnosing ARMS of the invention can be measured.

In one embodiment, the substance capable of binding to the biomarker for diagnosing ARMS of the invention is immobilized on a solid-support body. In one embodiment, any one of the substance capable of binding to biopyrrin, the substance capable of binding to cortisol, the substance capable of binding to a κFLC or a fragment thereof, the substance capable of binding to a λFLC or a fragment thereof, the substance capable of binding to creatinine, and/or the substance capable of binding to albumin is immobilized on a solid-support body. The solid-support body may be made of an appropriate material, such as plastics, e.g. polystyrene resin, polycarbonate resin, polyethylene resin, or polypropylene resin, or glass. The solid-support body may be a substrate in any shape such as a dish-, well-, strip-, or chip-shape. The surface of the substrate may be coated and/or modified, optionally.

Examples of the substance capable of binding to biopyrrin include an anti-biopyrrin antibody, for example, the antibody contained in Biopyrrin ELISA kit (Metallogenics Co., Ltd). Alternatively, a person skilled in the art may easily prepare the anti-biopyrrin antibody. The anti-biopyrrin antibody may be derived from a human, mouse, rat, rabbit, or ovine.

Examples of the substance capable of binding to cortisol include an anti-cortisol antibody, for example, the antibody contained in DetectX CORTISOL Enzyme Immunoassay Kit (ARBOR ASSAYS Inc.). Alternatively, a person skilled in the art may easily prepare the anti-cortisol antibody. The anti-cortisol antibody may be derived from a human, mouse, rat, rabbit, or ovine.

Examples of the substance capable of binding to a κFLC or a fragment thereof include an anti-κFLC antibody, for example, the antibody contained in FREELITE κ chain (MBL Co., Ltd., Code No.: BS-LK016BD). Alternatively, a person skilled in the art may easily prepare the anti-κFLC antibody. The anti-κFLC antibody may be derived from a human, mouse, rat, rabbit, or ovine. The anti-κFLC antibody can be prepared by administering a polypeptide of the immunoglobulin κ chain C region or an antigen site thereof to a mammal, such as a rat or a rabbit. The anti-κFLC antibody may be prepared, for example, by administering the polypeptide comprising the amino acid sequence of any one of SEQ ID NOS: 1 to 5 or the polypeptide consisting of the amino acid sequence of any one of SEQ ID NOS: 1 to 5 to a mammal, such as a rat or a rabbit.

Examples of the substance capable of binding to a λFLC or a fragment thereof include an anti-λFLC antibody, for example, the antibody contained in FREELITE λ chain (MBL Co., Ltd., Code No.: BS-LK018BD). Alternatively, a person skilled in the art may easily prepare the anti-AFLC antibody. The anti-AFLC antibody may be derived from a human, mouse, rat, rabbit, or ovine. The anti-λFLC antibody can be prepared by administering a polypeptide of the immunoglobulin λ chain C region or an antigen site thereof to a mammal, such as a rat or a rabbit. The anti-κFLC antibody is prepared, for example, by administering the polypeptide comprising the amino acid sequence of SEQ ID NO:6 or the polypeptide consisting of the amino acid sequence of SEQ ID NO:6 to a mammal, such as a rat or a rabbit.

Examples of the substance capable of binding to creatinine include a compound that causes a color reaction with creatinine, for example, the compound contained in DetectX Creatinine detection Kit (ARBOR ASSAYS Inc.).

Examples of the substance capable of binding to albumin include an anti-albumin antibody, for example, the antibody contained in Albumin (Human) ELISA Kit (Cayman Chemical Company). Alternatively, a person skilled in the art may easily prepare the anti-albumin antibody. The anti-albumin antibody may be derived from a human, mouse, rat, rabbit, or ovine.

The kit for diagnosing ARMS of the invention may further comprise a reagent used in the assay for performing the detection of the biomarker for diagnosing ARMS of the invention, measurement of the amount of the biomarker for diagnosing ARMS of the invention, and/or comparison of the amount of the biomarker for diagnosing ARMS of the invention. Examples of the assay include, but not limited to, immunological assay (e.g., Dot blot assay), Western blotting, enzyme-linked immunosorbent assay (ELISA), sandwich ELISA, Immunoelectrophoresis, single radial immunodiffusion (SRID), radioimmunoassay (RIA), enzyme immunoassay (EIA), latex immunoassay (LIA), fluorescence immunoassay (FIA), color reaction, colorimetric assay, and the like. Furthermore, the kit for diagnosing ARMS of the invention may comprise a primary antibody or secondary antibody used in the above assay. The primary antibody and/or secondary antibody may be labeled with an optional labeling marker, such as an inorganic substance, coloring matter, fluorochrome, dye, pigment, color reagent, coloring reagent, immunostaining reagent, enzyme, or radioisotope.

The kit for diagnosing ARMS of the invention may further contain instructions for measuring the amount of the biomarker for diagnosing ARMS of the invention, information showing data regarding the amount of the biomarker for diagnosing ARMS of the invention in healthy subjects and the like. Furthermore, the kit for diagnosing ARMS of the invention may contain a device or instrument used for collecting a biological sample from a subject (e.g., a container such as a urinalysis cup or a syringe).

By using the kit for diagnosing ARMS of the invention, detecting the biomarker for diagnosing ARMS of the invention, measuring the amount of the biomarker for diagnosing ARMS of the invention, and/or comparing the amount of the biomarker for diagnosing ARMS of the invention, can be performed. By using the kit for diagnosing ARMS of the invention, the diagnosis of ARMS can be performed. The diagnosis of ARMS can be conducted according to the method for diagnosing ARMS of the invention. Moreover, by using the kit for diagnosing ARMS of the invention, data for diagnosing ARMS can be obtained. The method for obtaining data can be performed according to a method for obtaining data for diagnosing ARMS of the invention.

### (4. Method for diagnosing ARMS of the invention, and Method for obtaining data for diagnosing ARMS of the invention)

By using the biomarker for diagnosing ARMS of the invention or the kit for diagnosing ARMS of the invention, ARMS of a subject can be diagnosed or examined. Moreover, using the biomarker for diagnosing ARMS of the invention or the kit for diagnosing ARMS of the invention, data for diagnosing or examining ARMS of a subject can be obtained. In the method for diagnosing ARMS and the method for obtaining data for diagnosing ARMS of the invention (hereinafter, collectively referred to as "the method of the invention"), the amount of the biomarker for diagnosing ARMS of the invention in a biological sample may be used as an indicator. By the method of the invention, data necessary for the treatment, prevention, diagnosis, differentiation, or prognosis estimation of ARMS or psychiatric disorders can be obtained. Further, the method of the invention can provide information useful for determining the onset, possibility of onset, or prognosis after the development of ARMS or psychiatric disorder. Furthermore, based on the results obtained from a subject by the method of the invention, a treatment method or a treatment regimen can be decided for the subject. In addition, based on the results obtained by the method of the invention, a therapeutic agent for ARMS or a psychiatric disorder can be screened or decided. By using such a therapeutic agent, a subject with ARMS or a psychiatric disorder can be treated, prevented, or managed. Alternatively, using the method of the invention, primary screening of ARMS or psychiatric disorders can be performed in such as routine health checkup system. In addition, in the first contact of a patient in a mental health clinic, the screening of ARMS or psychiatric disorders can also be carried out.

In one embodiment, the method of the invention comprises a step of measuring the amount of the biomarker for diagnosing ARMS of the invention in a biological sample collected from a subject. In a particular embodiment, the method of the invention comprises a step of measuring one or more (e.g., 1, 2, 3, 4, 5, or 6) amounts selected from the group consisting of the amount of biopyrrin, the amount of cortisol, the amount of an immunoglobulin free κ chain (κFLC) or a fragment thereof, and the amount of an immunoglobulin free λ chain (λFLC) or a fragment thereof in a biological sample collected from a subject.

In a preferred embodiment, the method of the invention comprises a step of measuring the amount of biopyrrin in a biological sample collected from a subject. In other embodiments, the method of the invention comprises a step of measuring the amount of cortisol in a biological sample collected from a subject. In further another embodiment, the method of the invention comprises a step of measuring the amount of a κFLC or a fragment thereof in a biological sample collected from a subject. In furthermore another embodiment, the method of the invention comprises a step of measuring the amount of a λFLC or a fragment thereof in a biological sample collected from a subject.

In a preferred embodiment, the method of the invention comprises a step of measuring the amount of biopyrrin; and one or more (e.g., 1, 2, 3, 4, 5 or 6) amounts selected from the group consisting of the amount of cortisol, the amount of a κFLC or a fragment thereof, and the amount of a λFLC or a fragment thereof in a biological sample collected from a subject. In another embodiment, the method of the invention comprises a step of measuring the amount of cortisol; and one or more (e.g., 1, 2, 3, 4, 5 or 6) amounts selected from the group consisting of the amount of biopyrrin, the amount of a κFLC or a fragment thereof, and the amount of a λFLC or a fragment thereof in a biological sample collected from a subject. In further another embodiment, the method of the invention comprises a step of measuring the amount of a κFLC or a fragment thereof; and one or more (e.g., 1, 2, 3, 4, 5 or 6) amounts selected from the group consisting of the amount of biopyrrin, the amount of cortisol, and the amount of a λFLC or a fragment thereof in a biological sample collected from a subject. In furthermore another embodiment, the method of the invention comprises a step of measuring the amount of a λFLC or a fragment thereof; and one or more (e.g., 1, 2, 3, 4, 5 or 6) amounts selected from the group consisting of the amount of biopyrrin, the amount of cortisol, and the amount of a κFLC or a fragment thereof in a biological sample collected from a subject.

In one embodiment, the method of the invention comprises a step of measuring the amount of biopyrrin and the amount of cortisol in a biological sample collected from a subject. In a preferred embodiment, the method of the invention comprises a step of measuring the amount of biopyrrin and the amount of a κFLC or a fragment thereof in a biological sample collected from a subject. In other preferred embodiments, the method of the invention comprises a step of measuring the amount of biopyrrin and the amount of a λFLC or a fragment thereof in a biological sample collected from a subject. In another embodiment, the method of the invention comprises a step of measuring the amount of cortisol and the amount of a κFLC or a fragment thereof in a biological sample collected from a subject. In further another embodiment, the method of the invention comprises a step of measuring the amount of cortisol and the amount of a λFLC or a fragment thereof in a biological sample collected from a subject. In furthermore another embodiment, the method of the invention comprises a step of measuring the amount of a κFLC or a fragment thereof and the amount of a λFLC or a fragment thereof in a biological sample collected from a subject.

In one embodiment, the method of the invention comprises a step of measuring the amount of biopyrrin, the amount of cortisol, and the amount of a κFLC or a fragment thereof in a biological sample collected from a subject. In another embodiment, the method of the invention comprises a step of measuring the amount of biopyrrin, the amount of cortisol, and the amount of a λFLC or a fragment thereof in a biological sample collected from a subject. In a preferred embodiment, the method of the invention comprises a step of measuring the amount of biopyrrin, the amount of a κFLC or a fragment thereof, and the amount of a λFLC or a fragment thereof in a biological sample collected from a subject. In further another embodiment, the method of the invention comprises a step of measuring the amount of cortisol, the amount of a κFLC or a fragment thereof, and the amount of a λFLC or a fragment thereof in a biological sample collected from a subject. In furthermore another embodiment, the method of the invention comprises a step of measuring the amount of biopyrrin, the amount of cortisol, the amount of a κFLC or a fragment thereof, and the amount of a λFLC or a fragment thereof in a biological sample collected from a subject.

The method of the invention may further comprise a step of measuring one or more amounts selected from the group consisting of the amount of creatinine and the amount of albumin in the biological sample collected from the subject. In one embodiment, the method of the invention further comprises a step of measuring the amount of creatinine in a biological sample collected from a subject. In another embodiment, the method of the invention further comprises a step of measuring the amount of albumin in the biological sample collected from the subject. In furthermore another embodiment, the method of the invention further comprises a step of measuring the amount of creatinine and the amount of albumin in the biological sample collected from the subject.

The measurement step can be carried out by using the kit for diagnosing ARMS of the invention. The measurement step can be carried out by using the substance capable of binding to the biomarker for diagnosing ARMS of the invention. In one embodiment, the measurement step comprises a step of contacting a biological sample collected from a subject with the substance capable of binding to the biomarker for diagnosing ARMS of the invention. The measurement step can be carried out by a common method known to a person skilled in the art. Examples of the common method known to a person skilled in the art include, but not limited to, immunological assay (e.g., Dot blot assay), Western blotting, enzyme-linked immunosorbent assay (ELISA), sandwich ELISA, immunoelectrophoresis, single radial immunodiffusion (SRID), radioimmunoassay (RIA), enzyme immunoassay (EIA), latex immunoassay (LIA), fluorescence immunoassay (FIA) and the like. Alternatively the step of measuring may be performed by using a commercial antibody or measurement kit. Examples of the commercial antibody or measurement kit include Biopyrrin ELISA kit (Metallogenics Co., Ltd), DetectX CORTISOL Enzyme Immunoassay Kit (ARBOR ASSAYS Inc.), FREELITE κ chain (MBL Co., Ltd., Code No.: BS-LK016BD), FREELITE λ chain (MBL Co., Ltd., Code No.: BS-LK018BD), DetectX Creatinine detection Kit (ARBOR ASSAYS Inc.) and the like.

The method of the invention may further comprise, a step of collecting a biological sample from a subject before the measurement step. The step may be performed by a method known to a person skilled in the art, but not limited thereto. For example, when the biological sample is urine, the step can be performed by using a container such as a urinalysis cup. The collected urine may further be subjected to a concentration or separation operation. Alternatively, for example, when the biological sample is blood, the step may be performed using a syringe or the like. When the biological sample is blood, the method of the invention may further comprise a step of obtaining serum or plasma from the collected blood.

The method of the invention further comprises a step of comparing any one of the amount of biopyrrin, the amount of cortisol, the amount of a κFLC or a fragment thereof, and/or the amount of a λFLC or a fragment thereof measured as above with a control. The method of the invention may further comprise a step of comparing the amount of albumin and/or the amount of creatinine measured as above with a control.

The method of the invention may further comprise a step of correcting the amount of biopyrrin, the amount of cortisol, the amount of a κFLC or a fragment thereof, and/or the amount of a λFLC or a fragment thereof in a biological sample collected from a subject, with the amount of creatinine in the biological sample collected from the subject. By the correction with the amount of creatinine, a value reflecting the renal function of the subject can be obtained. By comparing the corrected value with that of a control, ARMS can be diagnosed with higher accuracy. In one embodiment, the amount of biopyrrin is corrected with the amount of creatinine. In another embodiment, the amount of cortisol is corrected with the amount of creatinine. In further another embodiment, the amount of a κFLC or a fragment thereof is corrected with the amount of creatinine. In furthermore another embodiment, the amount of a λFLC or a fragment thereof is corrected with the amount of creatinine. The method of the invention may further comprise a step of correcting the amount of albumin in a biological sample collected from a subject with the amount of creatinine in the biological sample collected from the subject. A ratio of the amount of albumin to the amount of creatinine (ratio of albumin/creatinine) can be used in concluding whether the renal function of the subject is normal or not. In one embodiment, the correction is a division, namely calculating a ratio of an amount value to the amount of creatinine. A value reflecting the renal function of a subject can be obtained by this correcting step. The method of the invention may further comprise a step of comparing the corrected value with that of a control.

In the method of the invention, a biological sample is preferably blood or urine, more preferably, urine. In the method of the invention, a subject and a control are preferably a human. The age of the human is, preferably, a juvenile (boy or girl) or a sub-adult, more preferably, about 5 to about 25 years old, about 10 to about 20 years old, about 12 to about 18 years old, or about 14 to about 16 years old.

In a specific embodiment, the method of the invention comprises; (a) a step of measuring one or more (e.g., 1, 2, 3, 4, 5, or 6) amounts selected from the group consisting of the amount of biopyrrin, the amount of cortisol, the amount of an immunoglobulin free κ chain (κFLC) or a fragment thereof, and the amount of an immunoglobulin free λ chain (λFLC) or a fragment thereof in a biological sample collected from a subject; and (b) a step of comparing the amount measured in step (a) with that of a control.

In another specific embodiment, the method of the invention comprises; (a) a step of measuring one or more (e.g., 1, 2, 3, 4, 5, or 6) amounts selected from the group consisting of the amount of biopyrrin, the amount of cortisol, the amount of an immunoglobulin free κ chain (κFLC) or a fragment thereof, and the amount of an immunoglobulin free λ chain (λFLC) or a fragment thereof in a biological sample collected from a subject; and (b) a step of measuring one or more amounts selected from the group consisting of the amount of creatinine and the amount of albumin in the biological sample. In this embodiment, the method of the invention may further comprise (c) a step of comparing the amounts measured in steps (a) and (b) with these of a control.

In another specific embodiment, the method of the invention comprises (a) a step of measuring one or more (e.g., 1, 2, 3, 4, 5, or 6) amounts selected from the group consisting of the amount of biopyrrin, the amount of cortisol, the amount of an immunoglobulin free κ chain (κFLC) or a fragment thereof, and the amount of an immunoglobulin free λ chain (λFLC) or a fragment thereof in a biological sample collected from a subject; (b) a step of measuring the amount of creatinine in the biological sample; and (c) a step of correcting the amount measured in step (a) by using the amount measured in step (b). In this embodiment, the method of the invention may further comprise (d) a step of comparing the corrected amount with that of a control. The step of correcting (c) may comprise a step of dividing the amount measured in step (a) with the amount measured in step (b), namely calculating a ratio value of the amount measured in step (a) to the amount of creatinine measured in step (b).

In further another specific embodiment, the method of the invention comprises; (a) a step of measuring one or more (e.g., 1, 2, 3, 4, 5, or 6) amounts selected from the group consisting of the amount of biopyrrin, the amount of cortisol, the amount of an immunoglobulin free κ chain (κFLC) or a fragment thereof, and the amount of an immunoglobulin free λ chain (λFLC) or a fragment thereof in a biological sample collected from a subject; (b) a step of measuring the amount of albumin in the biological sample; (c) a step of measuring the amount of creatinine in the biological sample; and (d) a step of correcting the amounts measured in steps (a) and (b) by using the amount measured in step (c). In this embodiment, the method of the invention may further comprise (e) a step of comparing the corrected amounts with a control. The step of correcting (d) may comprise a step of dividing the amounts measured in step (a) and step (b) with the amount measured in step (c), namely calculating a ratio value of each of the amounts measured in step (a) and step (b) to the amount of creatinine measured in step (c).

The method of the invention can further comprise a step of determining whether or not a subject is ARMS or potential ARMS. In one embodiment, when the amount of biopyrrin in a biological sample collected from a subject increases compared with that of a control or is larger than the median value of a control group, the subject can be determined as ARMS or potential ARMS. In another embodiment, when the amount of biopyrrin in a biological sample collected from a subject increases compared with that of a control or is larger than the median value of a control group, the subject can be determined as ARMS or potential ARMS. In further another embodiment, when the amount of a κFLC or a fragment thereof in a biological sample collected from a subject decreases compared with that of a control or is smaller than the median value of a control group, the subject can be determined as ARMS or potential ARMS. In furthermore another embodiment, when the amount of a λFLC or a fragment thereof in a biological sample collected from a subject decreases compared with that of a control or is smaller than the median value of a control group, the subject can be determined as ARMS or potential ARMS.

In one embodiment, when both the amount of biopyrrin and the amount of cortisol in a biological sample collected from a subject increase compared with each one of a control or are larger than each median values of a control group, the subject can be determined as ARMS or potential ARMS. In a preferred embodiment, when the amount of biopyrrin in a biological sample collected from a subject increases compared with that of a control or is larger than the median value of a control group, as well as when the amount of a κFLC or a fragment thereof in the biological sample collected from the subject decreases compared with that of the control or is smaller than the median value of the control group, the subject can be determined as ARMS or potential ARMS. In a further preferred embodiment, when the amount of biopyrrin in a biological sample collected from a subject increases compared with that of a control or is larger than the median value of a control group, as well as when the amount of a λFLC or a fragment thereof in the biological sample collected from the subject decreases compared with that of the control or is smaller than the median value of the control group, the subject can be determined as ARMS or potential ARMS. In other embodiments, when the amount of cortisol in a biological sample collected from a subject increases compared with that of a control or is larger than the median value of a control group, as well as when the amount of a κFLC or a fragment thereof in the biological sample collected from the subject decreases compared with that of the control or is smaller than the median value of the control group, the subject can be determined as ARMS or potential ARMS. In another embodiment, when the amount of cortisol in a biological sample collected from a subject increases compared with that of a control or is larger than the median value of a control group, as well as when the amount of a λFLC or a fragment thereof in the biological sample collected from the subject decreases compared with that of the control or is smaller than the median value of the control group, the subject can be determined as ARMS or potential ARMS. In further another embodiment, when both of the amount of a κFLC or a fragment thereof and the amount of a λFLC or a fragment thereof in a biological sample collected from a subject decrease compared with each one of a control or are smaller than each median values of a control group, the subject can be determined as ARMS or potential ARMS.

In one embodiment, when both of the amount of biopyrrin and the amount of cortisol in a biological sample collected from a subject increase compared with each one of a control or are larger than each median values of a control group, as well as when the amount of a κFLC or a fragment thereof in the biological sample collected from the subject decreases compared with that of the control or is smaller than the median value of the control group, the subject can be determined as ARMS or potential ARMS. In other embodiments, when both the amount of biopyrrin and the amount of cortisol in a biological sample collected from a subject increase compared with each one of a control or are larger than each median values of a control group, as well as when the amount of a λFLC or a fragment thereof in the biological sample collected from the subject decreases compared with that of the control or is smaller than the median value of the control group, the subject can be determined as ARMS or potential ARMS. In a preferred embodiment, when the amount of biopyrrin in a biological sample collected from a subject increases compared with that of a control or is larger than the median value of a control group, as well as when both of the amount of a κFLC or a fragment thereof and the amount of a λFLC or a fragment thereof in the biological sample collected from the subject decrease compared with each one of the control or are smaller than each median values of the control group, the subject can be determined as ARMS or potential ARMS. In another embodiment, when the amount of cortisol in a biological sample collected from a subject increases compared with that of a control or is larger than the median value of a control group, as well as both of the amount of a κFLC or a fragment thereof and the amount of a λFLC or a fragment thereof in the biological sample collected from the subject decrease compared with each one of the control or are smaller than each median values of the control group, the subject can be determined as ARMS or potential ARMS. In further another embodiment, when both the amount of biopyrrin and the amount of cortisol increase compared with each one of a control or are larger than each median values of a control group, and as well as both of the amount of a κFLC or a fragment thereof and the amount of a λFLC or a fragment thereof in the biological sample collected from the subject decrease compared with each one of these from the control or are smaller than each median values of the control group, the subject can be determined as ARMS or potential ARMS.

A subject having an abnormal renal function may show an increase in the amount of biopyrrin, an increase in the amount of cortisol, a decrease in the amount of a κFLC or a fragment thereof, and/or a decrease in the amount of a λFLC or a fragment thereof as well as a change (increase or decrease) in the amount of creatinine and/or albumin. In contrast, an ARMS patient having a normal renal function may show an increase in the amount of biopyrrin, an increase in the amount of cortisol, a decrease in the amount of a κFLC or a fragment thereof, and/or a decrease in the amount of a λFLC or a fragment thereof, while showing no change in the amounts of creatinine and/or albumin. Therefore, in addition to the above determination criteria, when the amounts of creatinine and/or albumin in the biological sample collected from the subject show no change compared with these of the control or with the median value of the control group, the subject may also be determined as ARMS. Otherwise, in addition to the above determination criteria, when the albumin/creatinine ratio (ratio of the amount of albumin to the amount of creatinine) shows no change compared with that of a control or with the median value of a control group, or is not more than about 5, about 10, about 15, about 20, about 25, about 30, about 35, or about 40 mg/g, the subject can be determined as ARMS likewise.

By measuring the amount of creatinine and/or the amount of albumin in a biological sample collected from a subject, a determination may be made whether the renal function of the subject is normal or not. For example, when the amount of creatinine and/or the amount of albumin in a biological sample collected from a subject show no change compared with that of a control or with the median value of a control group, the renal function of the subject can be determined as normal. Alternatively, the ratio of albumin/creatinine shows no change compared with that of a control or with the median value of a control group, or is not more than about 5, about 10, about 15, about 20, about 25, about 30, about 35, or about 40 mg/g, the renal function of the subject can be determined as normal. Further, by measuring the amount of creatinine and/or the amount of albumin in a biological sample collected from a subject, a determination can be made whether or not a change in any one of the amount of biopyrrin, the amount of cortisol, the amount of a κFLC or a fragment thereof, and/or the amount of a λFLC or a fragment thereof is attributed to an abnormality of renal function of the subject. For example, when the amount of creatinine and/or the amount of albumin in a biological sample collected from the subject shows no change compared with that of a control or with the median value of a control group, a determination can be made that the change in any one of the amount of biopyrrin, the amount of cortisol, the amount of a κFLC or a fragment thereof, and/or the amount of a λFLC or a fragment thereof is not attributed to an abnormality of the renal function of the subject. Alternatively, when the ratio of albumin/creatinine shows no change compared with that of a control or with the median value of a control group, or is not more than about 5, about 10, about 15, about 20, about 25, about 30, about 35, or about 40 mg/g, a determination can be made that the change in the amount of biopyrrin, the amount of cortisol, the amount of a κFLC or a fragment thereof, and/or the amount of a λFLC or a fragment thereof is not attributed to an abnormality of renal function of the subject.

In another particular embodiment, each of the amount of biopyrrin, the amount of cortisol, the amount of a κFLC or a fragment thereof, the amount of a λFLC or a fragment thereof, and/or the amount of albumin in a biological sample collected from a subject is indicated as a value that is divided with the amount of creatinine in the biological sample collected from the subject, namely a ratio value to the amount of creatinine.

The above described "increase" may mean increasing to, e.g. about 1.3-fold, about 1.4-fold, about 1.5-fold, about 2-fold, about 3-fold, about 4-fold, about 5-fold, about 10-fold, about 15-fold, about 20-fold, about 30-fold, about 50-fold, about 100-fold or more. The above described "larger than the median value of a control group" may mean, e.g. about 1.3-fold, about 1.4-fold, about 1.5-fold, about 2-fold, about 3-fold, about 4-fold, about 5-fold, about 10-fold, about 15-fold, about 20-fold, about 30-fold, about 50-fold, about 100-fold, or more larger than the median value of a control group. The above described "decrease" may mean decreasing to, e.g. about 1.3-fold, about 1.4-fold, about 1.5-fold, about 2-fold, about 3-fold, about 4-fold, about 5-fold, about 10-fold, about 15-fold, about 20-fold, about 30-fold, about 50-fold, about 100-fold, or more. The above described "smaller than the median value of a control group" may mean, e.g. about 1.3-fold, about 1.4-fold, about 1.5-fold, about 2-fold, about 3-fold, about 4-fold, about 5-fold, about 10-fold, about 15-fold, about 20-fold, about 30-fold, about 50-fold, about 100-fold, or more smaller than the median value of a control group. The median value of a control group may be a median value known in the art or a value calculated based on an actually measured control group.

As used herein, "not change" or "no change" does not mean exactly the same before and after. The term may comprehend changes acceptable to a person skilled in the art, such as a change within about 40 %, about 35 %, about 30 %, about 25 %, about 20 %, about 15 %, about 10 %, about 5 %, or about 1 % or less.

### [EXAMPLE]

### (5.EXAMPLE)

Examples of the invention will be described below. The following Examples are described for a better understanding of the scope of claims of the invention, and not intended to limit the scope of claims of the invention.

### (EXAMPLE 1)

After receiving the approval of the Clinical Research Ethics Committee of Shimane University, spot urine samples were collected from patients who were diagnosed with at-risk mental state (ARMS) by a professional psychiatrist and from healthy subjects. The background characteristics of examinees are as shown in Table 1 below.

**[Table 1]**

| | Healthy subjects | ARMS patients | t-test (P value) |
|---|---|---|---|
| Examinee numbers | 21 | 19 | |
| Gender ratio (Male: Female) | 6:15 | 6:13 | 1 |
| Age (years) | 13. 04±4. 1 | 15. 05±2. 52 | 0.068 |

Found no significant differences in both of Fisher's exact test for Gender ratio and Student's t-test for Age.

Each of the amounts of albumin, creatinine, biopyrrin, cortisol, κFLC, and λFLC in the collected urine sample was measured respectively by using each commercially available measurement kit shown below. Each measurement was performed according to instructions of the measurement kit thereof.

**[Table 2]**

| Molecule to be Measured | Measurement kit Name (Company Name) |
|---|---|
| Albumin | Albumin (Human) ELISA Kit (Cayman Chemical Company) |
| Creatinine | DetectX Creatinine detection Kit (ARBOR ASSAYS Inc.) |
| Biopyrrin | Biopyrrin ELISA kit (Metallogenics Co., Ltd) |
| Cortisol | DetectX CORTISOL Enzyme Immunoassay Kit (ARBOR ASSAYS Inc.) |
| κFLC | FREELITE κ chain (MBL Co., Ltd.) |
| λFLC | FREELITE λ chain (MBL Co., Ltd.) |

Data regarding biopyrrin are shown below.

**[Table 3]**

| | healthy subjects | ARMS patients | t-test (P value) |
|---|---|---|---|
| Biopyrrin (µ mol/ml) | 3.61±2.81 | 5.39±2.55 | 0.043* |

| | | | |
|---|---|---|---|
| Performed by student's t-test *P<0.05, **P<0.001 | | | |

In order to reflect the renal functions of healthy subjects and ARMS patients, each amount was corrected (divided) with the amount of creatinine and the corrected values were compared. The results are shown in Table 4 below. As is clear from Table 4, significant differences were observed in the amounts of biopyrrin, cortisol, κFLC, and λFLC.

**[Table 4]**

| | healthy subjects | ARMS patients | t-test (P-value) |
|---|---|---|---|
| Albumin/Creatinine (mg/g) | 12.96±7.09 | 10.81±7.48 | 0.357 |
| Biopyrrin/Creatinine (µ mol/g) | 3.08±1.28 | 4.26±1.26 | 0.0058** |
| Cortisol/Creatinine (µ g/g) | 128.4±122.1 | 210.4±122.6 | 0.041* |
| κFLC/Creatinine (µ g/g) | 4.16±3.59 | 1.6±1.5 | 0.0058** |
| λFLC/Creatinine (µ g/g) | 1.33±1.15 | 0.57±0.41 | 0.0089** |

| | | | |
|---|---|---|---|
| Performed by student's t-test *P<0.05, **P<0.001 | | | |

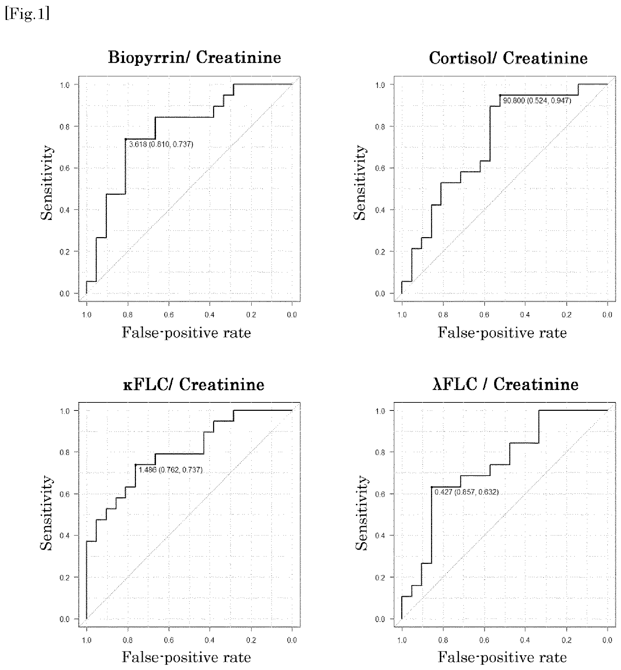

ROC curve analysis was performed on four factors that showed significant differences. Statistical software EZR (EASY R) was used for the analysis. The results are shown in Figure 1 and Table 5 below. Each of the four factors had an AUC (Area Under the Curve) of 0.7 or more, showing preferable results as a diagnostic method for ARMS. This indicates that biopyrrin, cortisol, κFLC and λFLC can be used, even if alone, as a biomarker for diagnosing ARMS.

**[Table 5]**

| | AUC | 95 % CI |
|---|---|---|
| Biopyrrin/Creatinine | 0.779 | 0.631 - 0.928 |
| Cortisol/Creatinine | 0.727 | 0.566 - 0.888 |
| κFLC/Creatinine | 0.799 | 0.662 - 0.937 |
| λFLC/Creatinine | 0.737 | 0.579 - 0.895 |

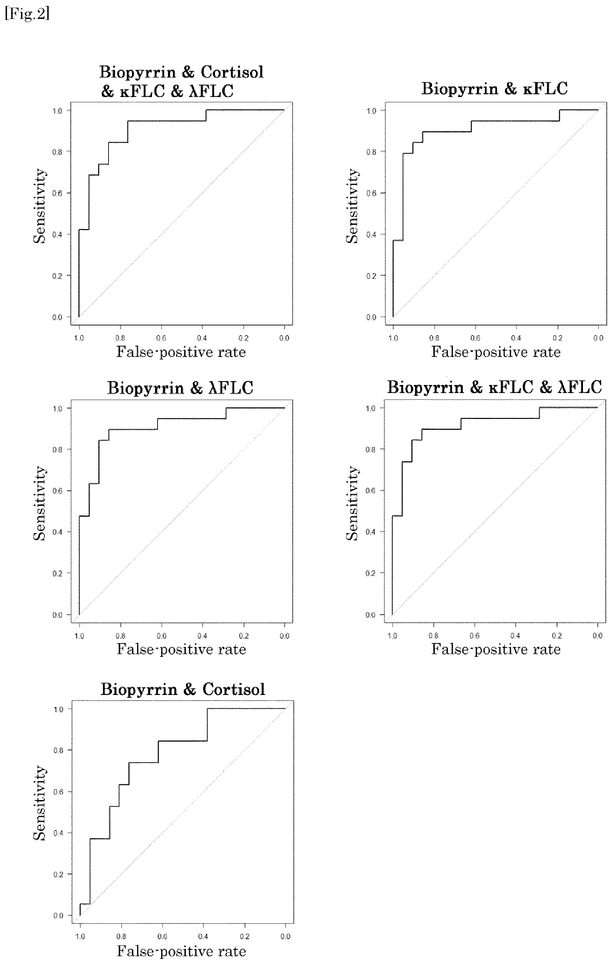

Further, by performing a logistic regression analysis of these four factors that showed significant differences, the data were integrated and analyzed. The statistical software EZR (EASY R) was used for the analysis. The results are shown in Figure 2 and Table 6 below. With respect to "biopyrrin & cortisol", the AUC was 0.7 or more. With respect to "biopyrrin & cortisol & κFLC & λFLC", "biopyrrin & κFLC", "biopyrrin & λFLC" and "biopyrrin & κFLC & λFLC", the AUCs were 0.9 or more. In all cases, the diagnostic probabilities were high. Among them, the combination of biopyrrin, κFLC and λFLC was found to have the highest diagnostic probability (AUC = 0.915).

**[Table 6]**

| | AUC | 95 % Confidence Interval |
|---|---|---|
| Biopyrrin & Cortisol & κFLC & λFLC | 0.91 | 0.819 - 1 |
| Biopyrrin & κFLC | 0.905 | 0.801 - 1 |
| Biopyrrin & λFLC | 0.907 | 0.811 - 1 |
| Biopyrrin & κFLC & λFLC | 0.915 | 0.822 - 1 |
| Biopyrrin & Cortisol | 0.779 | 0.634 - 0.925 |

### [Sequence Listing Free Text]

SEQ ID NO:1 shows an amino acid sequence of the constant region of κFLC (human-derived).
SEQ ID NO:2 shows an amino acid sequence of an antigen site of κFLC (human-derived).
SEQ ID NO:3 shows an amino acid sequence of an antigen site of κFLC (human-derived).
SEQ ID NO:4 shows an amino acid sequence of the constant region of κFLC (rat-derived).
SEQ ID NO:5 shows an amino acid sequence of an antigen site of κFLC (rat-derived).
SEQ ID NO:6 shows an amino acid sequence of the constant region of AFLC (human-derived).

## Claims

1. A biomarker for diagnosing at-risk mental state (ARMS), comprising one or more selected from the group consisting of biopyrrin, cortisol, an immunoglobulin free κ chain (κFLC) or a fragment thereof, and an immunoglobulin free λ chain (λFLC) or a fragment thereof.

2. The biomarker for diagnosing ARMS according to claim 1, comprising biopyrrin.

3. The biomarker for diagnosing ARMS according to claim 1 or 2, comprising biopyrrin; and one or more selected from the group consisting of cortisol, a κFLC or a fragment thereof, and a λFLC or a fragment thereof.

4. The biomarker for diagnosing ARMS according to any one of claims 1 to 3, comprising biopyrrin and cortisol.

5. The biomarker for diagnosing ARMS according to any one of claims 1 to 3, comprising biopyrrin and a κFLC or a fragment thereof.

6. The biomarker for diagnosing ARMS according to any one of claims 1 to 3, comprising biopyrrin and a λFLC or a fragment thereof.

7. The biomarker for diagnosing ARMS according to any one of claims 1 to 3, comprising biopyrrin, a κFLC or a fragment thereof, and a λFLC or a fragment thereof.

8. The biomarker for diagnosing ARMS according to any one of claims 1 to 3, comprising biopyrrin, cortisol, a κFLC or a fragment thereof, and a λFLC or a fragment thereof.

9. The biomarker for diagnosing ARMS according to any one of claims 1 to 8, further comprising one or more selected from the group consisting of creatinine and albumin.

10. A kit for diagnosing at-risk mental state (ARMS), comprising one or more substances selected from the group consisting of a substance capable of binding to biopyrrin, a substance capable of binding to cortisol, a substance capable of binding to an immunoglobulin free κ chain (κFLC) or a fragment thereof, and a substance capable of binding to an immunoglobulin free λ chain (λFLC) or a fragment thereof.

11. The kit for diagnosing ARMS according to claim 10, comprising a substance capable of binding to biopyrrin.

12. The kit according to claim 10 or 11, comprising a substance capable of binding to biopyrrin; and one or more substances selected from the group consisting of a substance capable of binding to cortisol, a substance capable of binding to a κFLC or a fragment thereof, and a substance capable of binding to a λFLC or a fragment thereof.

13. The kit according to any one of claims 10 to 12, comprising a substance capable of binding to biopyrrin and a substance capable of binding to cortisol.

14. The kit according to any one of claims 10 to 12, comprising a substance capable of binding to biopyrrin and a substance capable of binding to a κFLC or a fragment thereof.

15. The kit according to any one of claims 10 to 12, comprising a substance capable of binding to biopyrrin and a substance capable of binding to a λFLC or a fragment thereof.

16. The kit according to any one of claims 10 to 12, comprising a substance capable of binding to biopyrrin, a substance capable of binding to a κFLC or a fragment thereof, and a substance capable of binding to a λFLC or a fragment thereof.

17. The kit according to any one of claims 10 to 12, comprising a substance capable of binding to biopyrrin, a substance capable of binding to cortisol, a substance capable of binding to a κFLC or a fragment thereof, and a substance capable of binding to a λFLC or a fragment thereof.

18. The kit according to any one of claims 10 to 17, further comprising one or more substances selected from the group consisting of a substance capable of binding to creatinine and a substance capable of binding to albumin.

19. The kit according to any one of claims 10 to 18, wherein the substance is an antibody or an organic compound.

20. The kit according to any one of claims 10 to 19, wherein the substance is immobilized on a solid-support body.

21. A method for obtaining data for diagnosing at-risk mental state (ARMS) in a subject, comprising a step of measuring one or more amounts selected from the group consisting of the amount of biopyrrin, the amount of cortisol, the amount of an immunoglobulin free κ chain (κFLC) or a fragment thereof, and the amount of an immunoglobulin free λ chain (λFLC) or a fragment thereof in a biological sample collected from the subject.

22. The method according to claim 21, comprising a step of measuring the amount of biopyrrin in the biological sample.

23. The method according to claim 21 or 22, comprising a step of measuring the amount of biopyrrin; and one or more amounts selected from the group consisting of the amount of cortisol, the amount of a κFLC or a fragment thereof, and the amount of a λFLC or a fragment thereof in the biological sample.

24. The method according to any one of claims 21 to 23, comprising a step of measuring the amount of biopyrrin and the amount of cortisol in the biological sample.

25. The method according to any one of claims 21 to 23, comprising a step of measuring the amount of biopyrrin and the amount of a κFLC or a fragment thereof in the biological sample.

26. The method according to any one of claims 21 to 23, comprising a step of measuring the amount of biopyrrin and the amount of a λFLC or a fragment thereof in the biological sample.

27. The method according to any one of claims 21 to 23, comprising a step of measuring the amount of biopyrrin, the amount of a κFLC or a fragment thereof, and the amount of a λFLC or a fragment thereof in the biological sample.

28. The method according to any one of claims 21 to 23, comprising a step of measuring the amount of biopyrrin, the amount of cortisol, the amount of a κFLC or a fragment thereof, and the amount of a λFLC or a fragment thereof in the sample.

29. The method according to any one of claims 21 to 28, further comprising a step of measuring the amount of albumin in the biological sample.

30. The method according to any one of claims 21 to 29, further comprising a step of measuring the amount of creatinine in the biological sample.

31. The method according to any one of claims 21 to 30, comprising a step of comparing the amount with that of a control.

32. The method according to any one of claims 21 to 29, further comprising a step of calculating a ratio value of the amount to the amount of creatinine in the biological sample.

33. The method according to claims 32, comprising a step of comparing the ratio value with that of a control.

34. The method according to any one of claims 21 to 33, wherein the biological sample is urine.


### [Sequence Listing]

SEQUENCE LISTING

<110>  RRESVO Inc.

<120>  Biomerker for diagnosis of ARMS

<130>  0787002WO1

<160>  6

<170>  PatentIn version 3.5

<210>  1
<211>  106
<212>  PRT
<213>  Homo sapiens

<400>  1

Thr Val Ala Ala Pro Ser Val Phe Ile Phe Pro Pro Ser Asp Glu Gln
1               5                   10                  15

Leu Lys Ser Gly Thr Ala Ser Val Val Cys Leu Leu Asn Asn Phe Tyr
            20                  25                  30

Pro Arg Glu Ala Lys Val Gln Trp Lys Val Asp Asn Ala Leu Gln Ser
        35                  40                  45

Gly Asn Ser Gln Glu Ser Val Thr Glu Gln Asp Ser Lys Asp Ser Thr
    50                  55                  60

Tyr Ser Leu Ser Ser Thr Leu Thr Leu Ser Lys Ala Asp Tyr Glu Lys
65                  70                  75                  80

His Lys Val Tyr Ala Cys Glu Val Thr His Gln Gly Leu Ser Ser Pro
                85                  90                  95

Val Thr Lys Ser Phe Asn Arg Gly Glu Cys
            100                 105

<210>  2
<211>  20
<212>  PRT
<213>  Homo sapiens

<400>  2

Ser Val Thr Glu Gln Asp Ser Lys Asp Ser Thr Tyr Ser Leu Ser Ser
1               5                   10                  15

Thr Leu Thr Leu
            20

<210>  3

<211>  21
<212>  PRT
<213>  Homo sapiens

<400>  3

Cys Glu Val Thr His Gln Gly Leu Ser Ser Pro Val Thr Lys Ser Phe
1               5                   10                  15

Asn Arg Gly Glu Cys
            20

<210>  4
<211>  106
<212>  PRT
<213>  Rattus norvegicus

<400>  4

Ala Asp Ala Ala Pro Thr Val Ser Ile Phe Pro Pro Ser Met Glu Gln
1               5                   10                  15

Leu Thr Ser Gly Gly Ala Thr Val Val Cys Phe Val Asn Asn Phe Tyr
            20                  25                  30

Pro Arg Asp Ile Ser Val Lys Trp Lys Ile Asp Gly Ser Glu Gln Arg
        35                  40                  45

Asp Gly Val Leu Asp Ser Val Thr Asp Gln Asp Ser Lys Asp Ser Thr
    50                  55                  60

Tyr Ser Met Ser Ser Thr Leu Ser Leu Thr Lys Val Glu Tyr Glu Arg
65                  70                  75                  80

His Asn Leu Tyr Thr Cys Glu Val Val His Lys Thr Ser Ser Ser Pro
                85                  90                  95

Val Val Lys Ser Phe Asn Arg Asn Glu Cys
            100                 105

<210>  5
<211>  20
<212>  PRT
<213>  Rattus norvegicus

<400>  5

Ser Val Thr Asp Gln Asp Ser Lys Asp Ser Thr Tyr Ser Met Ser Ser
1               5                   10                  15

Thr Leu Ser Leu
            20

<210>  6
<211>  94
<212>  PRT
<213>  Homo sapiens

<400>  6

Ala Pro Ser Val Thr Leu Phe Pro Pro Ser Ser Glu Glu Leu Gln Ala
1               5                   10                  15

Asn Lys Ala Thr Leu Val Cys Leu Ile Ser Asp Phe Tyr Pro Gly Ala
            20                  25                  30

Val Thr Val Ala Trp Lys Ala Asp Ser Ser Pro Val Lys Ala Gly Val
        35                  40                  45

Glu Thr Thr Thr Pro Ser Lys Gln Ser Asn Asn Lys Tyr Ala Ala Ser
    50                  55                  60

Ser Tyr Leu Ser Leu Thr Pro Glu Gln Trp Lys Ser His Lys Ser Tyr
65                  70                  75                  80

Ser Cys Gln Val Thr His Glu Gly Ser Thr Val Glu Lys Thr
                85                  90